# EUROPEAN PATENT APPLICATION

(11) **EP 2 944 303 A1**
(43) Date of publication of application: **18.11.2015**
(21) Application number: 15160469.1
(22) Date of filing: 24.03.2015
(51) Int. Cl.: A61K 8/368, A61K 8/81, A61Q 19/00, A61Q 19/10

(54) **AN EXFOLIATING ANTI-ACNE FACE CLEANSING COMPOSITION**

(30) Priority: 15.05.2014 US 201414278324
(71) Applicant: The Dial Corporation, Scottsdale, AZ 85255 (US)
(72) Inventor: Guerra, Kanani, AZ 85027, Phoenix, (US)
(74) Representative: Semrau, Markus

(57) **Abstract**

Methods and apparatus are provided for an exfoliating anti-acne face cleansing composition. The exfoliating anti-acne face cleansing composition includes an anti-acne active ingredient and exfoliating particles. The exfoliating anti-acne face cleansing composition further includes a high molecular weight polymer having a molecular weight at least equal to ten kiloDaltons (kD) and a moisturizer. A ratio of the high molecular weight polymer to total formula solids is between 8:100 and 12:100. A ratio of the high molecular weight polymer to an amount of moisturizer is between 4:10 and 9:10. A pH of the exfoliating anti-acne face cleansing composition is less than 5.0.

## Description

### FIELD OF THE INVENTION

The present invention generally relates to a face cleansing composition, and more particularly relates to an exfoliating anti-acne face cleansing composition that has moisturization and hydrating benefits.

### BACKGROUND OF THE INVENTION

Exfoliating facial scrubs that target acne-prone skin may include salicylic acid as the active ingredient. These scrubs are kept at a low pH to keep the salicylic acid in its acid form. These scrubs may also include a high level of surfactants in order to associate with the suspending aide and allow for the suspension of exfoliating particles. The inclusion of a large quantity of surfactants and suspending aides as well as the low pH make an exfoliating facial scrub prone to cause skin dryness, and may result in poor moisturization characteristics.

Accordingly, it is desirable to have an exfoliating anti-acne face cleansing composition which has reduced amounts of both surfactants and suspending aides, while providing moisturization benefits to a user. Furthermore, other desirable features and characteristics of the present invention will become apparent from the subsequent detailed description of the invention and the appended claims, taken in conjunction with the accompanying drawings and this background of the invention.

### BRIEF SUMMARY OF THE INVENTION

An exfoliating anti-acne face cleansing composition includes an anti-acne active ingredient, exfoliating particles, a high molecular weight polymer having a molecular weight at least equal to ten kiloDaltons (kD), and a moisturizer. A ratio of the high molecular weight polymer to total formula solids is between 8:100 and 12:100. A ratio of the high molecular weight polymer to the amount of moisturizer is between 4:10 and 9:10. A pH of the exfoliating anti-acne face cleansing composition is less than 5.0.

An exfoliating anti-acne face cleansing product includes a container, and an exfoliating anti-acne face cleansing composition disposed within the container. The face cleansing composition includes an anti-acne active ingredient, exfoliating particles, a high molecular weight polymer having a molecular weight at least equal to ten kiloDaltons (kD), a surfactant, a moisturizer, and an aqueous medium in which the anti-acne active ingredient, the exfoliating particles, the high molecular weight polymer, the surfactant, and the moisturizer are distributed. A ratio of the high molecular weight polymer to total formula solids is between 8:100 and 12:100. A ratio of the high molecular weight polymer to an amount of moisturizer is between 4:10 and 9:10. A pH of the exfoliating anti-acne face cleansing composition is less than 5.0.

An exfoliating anti-acne face cleansing composition includes salicylic acid, exfoliating particles, a surfactant, a cross-linked copolymer of acrylic acid, a cross-linked copolymer of methacrylic acid, or combinations thereof, and moisturizer. A ratio of the cross-linked copolymer of acrylic acid, the cross-linked copolymer of methacrylic acid, or combinations thereof to total formula solids is between 8:100 and 12:100. A ratio of the cross-linked copolymer of acrylic acid, the cross-linked copolymer of methacrylic acid, or combinations thereof to an amount of moisturizer is between 4:10 and 9:10. A pH of the exfoliating anti-acne face cleansing composition is less than 5.0.

### DETAILED DESCRIPTION OF THE INVENTION

The following detailed description of the invention is merely exemplary in nature and is not intended to limit the invention or the application and uses of the invention. Furthermore, there is no intention to be bound by any theory presented in the preceding background of the invention or the following detailed description of the invention.

Anti-acne face scrubs contain sufficient surfactants and suspending aides so as to stably suspend exfoliating particles within the scrubs. However, such amount of surfactants and suspending aides may make the scrubs prone to result in dry skin upon application. Such a reaction may lead to a less than satisfactory customer experience.

Accordingly, the principles described herein provide a composition which allows for effective acne prevention, all while providing moisturization benefits to the user. More specifically, the present specification describes an exfoliating anti-acne face cleansing composition that contains a high molecular weight polymer in a particular ratio to other components of the exfoliating anti-acne face cleansing composition. The high molecular weight polymer in this particular ratio acts as a suspension agent for the exfoliating particles and also acts as a film former to help deliver and deposit ingredients such as emollients, humectants, moisturizers, and other components to the skin. In other words, the high molecular weight polymer increases the efficacy of the emollients, humectants, moisturizers, exfoliating particles and other components.

The exfoliating anti-acne face cleansing composition described herein also provide other sensory benefits such as a high volume of lather, clean-rinsing, desirable application aesthetics, desirable post-application aesthetics, and increased skin smoothness.

The exfoliating anti-acne face cleansing composition includes an anti-acne active ingredient. As used in the present specification and in the appended claims, an anti-acne active ingredient may refer to any compound used to prevent, reduce, eliminate, or otherwise combat acne on a skin surface such as the face. For example, an anti-acne active ingredient may prevent acne from forming on the skin surface. An anti-acne active ingredient may also reduce acne currently present on the skin surface. An example of an anti-acne active ingredient is salicylic acid. In some examples, the anti-acne active ingredient forms from 0.01 to 2.50 weight percent of the exfoliating anti-acne face cleansing composition. For example, the anti-acne active ingredient may form 2.00 weight percent of the exfoliating anti-acne face cleansing composition.

The exfoliating anti-acne face cleansing composition includes a number of exfoliating particles. Exfoliating particles are solids suspended in the exfoliating anti-acne face cleansing composition. The solids act as an abrasive when the exfoliating anti-acne face cleansing composition is used to cleanse skin. The abrasive removes dead skin cells from the surface of the skin. Removal of the dead skin cells from the surface of the skin allows improved access and deeper penetration of the surfactants and any moisturizer which may be present in the exfoliating anti-acne face cleansing composition. This may improve both the feel and appearance of skin. The exfoliating particles may also use a chemical process to remove dead skin cells. A chemical exfoliator loosens bonds that hold cells together. Loosening these bonds allows the dead skin cells to be easily washed away. Examples of exfoliating particles include, but are not limited to, polyethylene, silica, alumina, fruit seeds, and plant seeds, among other exfoliating particles. The exfoliating particles may form from 0.01 to 10.00 weight percent of the exfoliating anti-acne face cleansing composition.

Examples of exfoliating particles which may be included in an exfoliating anti-acne face cleansing composition include actinidia chinensis (kiwi) seed, alumina, aluminum iron silicates, aluminum silicate, amethyst powder, amorphophallus konjac root powder, arachis hypogaea (peanut) flour, attapulgite, avena sativa (oat) bran, avena sativa (oat) kernel flour, avena sativa (oat) kernel meal, bambusa arundinacea stem powder, calcium carbonate, calcium phosphate, calcium pyrophosphate, calcium sulfate, carya illinoensis (pecan) shell powder, chalk, chitin, citrus tangerina (tangerine) peel, cocos nucifera (coconut) shell powder, colloidal oatmeal, conchiolin powder, coral powder, corylus avellana (hazel) shell powder, diamond powder, diatomaceous earth, dicalcium phosphate, dicalcium phosphate dihydrate, dolomite, egg shell powder, eijitsu, elguea clay, emerald, fragaria vesca (strawberry) seed, fuller's earth, glycine soja (soybean) flour, helianthus annuus (sunflower) seed meal, hordeum distichon (barley) seed flour, hordeum vulgare powder, hordeum vulgare seed flour, hydrated silica, hydroxyapatite, illite, juglans mandshurica (walnut) shell powder, juglans regia (walnut) shell powder, kaolin, kurumi kaku, lauryl acrylate/VA crosspolymer, lithothamnium calcarum powder, lithoammium corallioides powder, loess, luffa cylindrica fruit, magnesium potassium fluorosilicate, magnesium sodium fluorosilicate, magnesium trisilicate, melaleuca alternifolia leaf powder, microcrystalline cellulose, montmorillonite, moroccan lava clay, mother of pearl, myristyl betaine, oenothera biennis (evening primrose) seed, olea europaea (olive) fruit, olea europaea (olive) husk powder, olea europaea (olive) seed powder, oryza sativa (rice) bran, oryza sativa (rice) germ powder, oubaku, oyster shell powder, papaver so niferum seed, perlite, persea gratissima (avocado) fruit powder, phaseolus radiatus seed starch, platinum powder, polyethylene, potassium undecylenoyl glutamate, prunus amygdalus dulcis (sweet almond) seed meal, prunus amygdalus dulcis (sweet almond) shell powder, prunus armeniaca (apricot) seed powder, prunus mume fruit, prunus persica (peach) seed powder, pumice, quartz, rubus idaeus (raspberry) seed, salt mine mud, sand, sea salt, secale cereale (rye) seed flour, silica, sodium bicarbonate, sodium hydroxypropyl starch phosphate, sodium magnesium fluorosilicate, sodium silicoaluminate, symphytum officinale leaf powder, talc, theobroma cacao (cocoa) shell powder, tin oxide, titanium oxynitride, topaz, touki, tricalcium phosphate, triticum vulgare (wheat) bran, triticum vulgare (wheat) germ powder, triticum vulgare (wheat) kernel flour, triticum vulgare (wheat) starch, vaccinium angustifolium (blueberry) seed, vaccinium macrocarpon (cranberry) seed, volcanic ash, wood powder, yokuinin, zea mays (corn) cob meal, zea mays (corn) cob powder, zea mays (corn) kernel meal, zea mays (corn) seed flour, zea mays (corn) starch, zirconium silicate, or combinations thereof. While specific reference has been made to certain exfoliating particles, the number of exfoliating particles may include any type, combination, or mixture of exfoliating particles. For example, the number of exfoliating particles may be prunus persica seed powder. In another example, the number of exfoliating particles include dolomite, emerald, platinum powder, microcrystalline cellulose, and zea mays corn meal.

The exfoliating anti-acne face cleansing composition includes a high molecular weight polymer. As used in the present specification and in the appended claims, a high molecular weight polymer may be a polymer having a molecular weight of at least 10 kiloDaltons (kD). The high molecular weight polymer acts as a suspending agent for the exfoliating particles. In other words, via the high molecular weight polymer, the exfoliating particles are distributed across the skin to remove dead skin cells, either mechanically or chemically, from the skin surface. In other words, a suspending agent is a molecule which assists in the suspension of a solid particle in a solution. A suspending agent may thicken the composition, providing a gel-like material in which a solid is suspended. A suspending agent may be a polymer or copolymer, which is capable of suspending a number of solid particles in solution.

The high molecular weight polymer also acts as a film former to aid in the delivery of other ingredients such as emollients, humectants, moisturizers, and other anti-acne face cleansing composition components. In this example, the high molecular weight polymer may increase the efficacy of the emollients, humectants, moisturizers, and other anti-acne face cleansing composition components. In some examples, the high molecular weight polymer is a cross-linked copolymer of acrylic acid. The high molecular weight polymer may be a cross-linked copolymer of methacrylic acid. The high molecular weight polymer may be a simple ester of the cross-linked copolymers described above. More specifically, the high molecular weight polymer may be a C 1-C4 ester of the cross-linked copolymers. More detail concerning the amount of high molecular weight polymer included in the exfoliating anti-acne face cleansing composition is given below.

Examples of high molecular weight polymers that may be included in the exfoliating anti-acne face cleansing composition may include Acrylates/C10-30 Alkyl Acrylate Crosspolymer, Acrylates Copolymer , Acrylates Crosspolymer, Acrylates Crosspolymer-3, Acrylates Crosspolymer-4, Carbomer, Polyacrylate Crosspolymer-4 Polyacrylate Crosspolymer-6, Polyacrylate Crosspolymer-11, Polyacrylate Crosspolymer-14, Polyacrylic Acid, and Poly C10-30 Alkyl Acrylate, among other high molecular weight polymers.

The use of a high molecular weight polymer in the exfoliating anti-acne face cleansing composition is beneficial in that it improves application and post-application feel as well as provide high lather aesthetics and improved skin smoothness. Still further, the high molecular weight polymer increases the efficacy of other components such as exfoliating particles, emollients, humectants, and other moisturizers by being a suspending agent and film former for those components.

The exfoliating anti-acne face cleansing composition also includes moisturizer. As used in the present specification, the term moisturizer may refer to moisturizers, humectants, emollients, or other components that increase the water-content level of the skin surface upon application. Moisturizers may be chemical agents which cause the skin to be softer. Moisturizers may act by reducing evaporation from the surface of the skin. Humectants may be used as moisturizers. Oils may be used as moisturizers. Examples of moisturizers that may be included in the exfoliating anti-acne face cleansing composition may include ascorbic acid, ascorbyl dipalmitate, acetamide MEA, glucose glutamate, glucuronic acid, TEA-lactate, TEA-PCA, corn syrup, fructose, glucose, glycerin, glycol, 1 ,2,6-hexanetriol, sodium lactate, sodium PCA, hydrogenated starch hydrolysate, inositol, lactic acid, lactose, mannitol, PCA, PEG- 10 propylene glycol, polyamino sugar condensate, propylene glycol, pyridoxine dilaurate, saccharide hydrolysate, hydroxystearyl methylglucamine, glucamine, maltitol, mannitol, methyl gluceth-10, methyl gluceth-20, riboflavin, PEG-4, PEG-6, PEG-8, PEG-9, PEG- 10, PEG- 12, PEG- 14, PEG- 16, PEG- 18, PEG- 20, PEG-32, PEG-40, glutamic acid, glycereth-7, glycereth-12, glycereth-26, saccharide isomerate, sorbeth-20, sorbitol, sucrose, thioglycerin, tris-(hydroxymethyl)nitromethane, tromethamine, histidine, PEG-75, PEG-135, PEG-150, PEG-200, PEG-5 pentaerythritol ether, polyglyceryl sorbitol, sorbitol, urea, xylitol, and mixtures thereof. In some examples, the moisturizer may form between 1.00 and 10.00 weight percent of the exfoliating anti-acne face cleansing composition.

The exfoliating anti-acne face cleansing composition includes a surfactant. A surfactant has a hydrophobic end and a hydrophilic end. The hydrophobic end allows the surfactant to interact with uncharged molecules, such as oils. The hydrophilic end facilitates the interaction of the molecule with charged or polar molecules, such as water. The hydrophilic end is used to classify surfactants, which may be anionic, cationic, nonionic, or amphoteric. Anionic surfactants have a negatively charged hydrophilic end, which may be present as a sulfate, sulfonate, carboxylate, phosphate, or the like; anionic surfactants may be sensitive to water hardness. Cationic surfactants are those that have a positively charged hydrophilic end, such as a quaternary amine. Nonionic surfactants have a hydrophilic end which may be charge neutral, such as an ethoxylate, glycoside, or poly-ol; such surfactants may not be sensitive to water hardness. Amphoteric or zwitterionic surfactants may have both a positive and negative charge on their hydrophilic ends, such as amine oxides, sultaines, or betaines. In some examples, the surfactants include lauramidopropyl betaine, lauryl betaine, lauryl sultaine, lauryl sulfate, lauryl sulfonate, cocamidopropyl betaine, myristyl betaine, myristyl sultaine, myristyl sulfate, myristyl sulfonate, cetyl betaine, cetyl sultaine, cetyl sulfate, cetyl sulfonate, stearyl betaine, stearyl sultaine, stearyl sulfate, stearyl sulfonate, or combinations thereof. While specific reference has been made to certain surfactants, the number of surfactants in the exfoliating anti-acne face cleansing composition may include any type, combination or mixture of surfactants. The surfactant may form between 0.01 to 15.00 weight percent of the exfoliating anti-acne face cleansing composition.

The exfoliating anti-acne face cleansing composition may also include a number of other components. These components may include a pH adjuster, a fragrance, a die, an extract, or any other component that improves the customer experience.

The exfoliating anti-acne face cleansing composition may include an aqueous medium in which the anti-acne active ingredient, the exfoliating particle, the high molecular weight polymer, the surfactant, the moisturizer, other ingredients, or combinations thereof are distributed. The aqueous medium may be water. In some examples, the aqueous medium forms between 70 and 90 weight percent of the exfoliating anti-acne face cleansing composition. For example, the aqueous medium may form between 75 and 80 weight percent of the exfoliating anti-acne face cleansing composition.

In some examples, the exfoliating anti-acne face cleansing composition may be defined by the relative amounts of different components within the exfoliating anti-acne face cleansing composition. In general, the term total formula solids refers to components of the exfoliating anti-acne face cleansing composition that are not liquid. For example, the total formula solids may include all components within the exfoliating anti-acne face cleansing composition with the exception of the aqueous medium.

As stated above, the exfoliating anti-acne face cleansing composition may be defined by the relative amounts of different components within the exfoliating anti-acne face cleansing composition. For example, a ratio of the high molecular weight polymer to total formula solids may be between 8:100 and 12:100. Including the high molecular weight polymer in such a ratio is unique in that typical anti-acne scrubs may use significantly more or less high molecular weight polymer which may lead to a more complex composition or which may result in harsh and drying conditions described above. For example, some anti-acne face cleansing compositions have a ratio of high molecular weight polymer to total formula solids in a lesser amount such as 7:100. In some examples, a ratio of the high molecular weight polymer to total formula solids is between 9:100 and 11:100.

The exfoliating anti-acne face cleansing composition may also be defined by a ratio of the high molecular weight polymer to an amount of moisturizer. For example, the exfoliating anti-acne face cleansing composition may be defined by a ratio of the high molecular weight polymer to the number of moisturizers that may be between 4:10 and 9:10. In some examples, the ratio of high molecular weight polymer to the number of moisturizers is between 5:10 and 8:10. Including the high molecular weight polymer in such a ratio is unique in that many anti-acne face cleansing compositions include greater amounts of moisturizer, which may suggest a higher moisturizing effect. However, as will be demonstrated below, the exfoliating anti-acne face cleansing composition with a high molecular weight polymer to moisturizer ratio between 4:10 and 9:10 had more moisturization benefits as compared to face cleansing compositions with more moisturizer.

The exfoliating anti-acne face cleansing composition may also be defined by a ratio of the high molecular weight polymer to the surfactant. For example, the exfoliating anti-acne face cleansing composition may be defined by a ratio of the high molecular weight polymer to the surfactant that may be between 1:10 and 2:10. In one example, the ratio of high molecular weight polymer to the surfactant is between 1.25:10 and 1.75:10.

The exfoliating anti-acne face cleansing composition may also be defined by a ratio of the surfactant to an amount of moisturizer. For example, the exfoliating anti-acne face cleansing composition may be defined by a ratio of the surfactant to the amount of moisturizer that may be between 30:10 and 50:10. In one example, the ratio of surfactant to the amount of moisturizers is between 40:10 and 45:10.

The exfoliating anti-acne face cleansing composition may also be defined by a ratio of the total formula solids to the amount of moisturizer. For example, the exfoliating anti-acne face cleansing composition may be defined by a ratio of the total formula solids to the amount of moisturizer that may be between 60:10 and 80:10. In one example, the ratio of total formula solids to the number of moisturizers is between 65:10 and 80:10. Including the moisturizer in such a ratio is unique in that many anti-acne face cleansing compositions include greater amounts of moisturizer, which may suggest a higher moisturizing effect. However, as will be demonstrated below, the exfoliating anti-acne face cleansing composition with a total formula solids to moisturizer ratio between 60:10 and 80:10 had more moisturization benefits as compared to face cleansing compositions with more moisturizer.

In some examples, the exfoliating anti-acne face cleansing composition may be defined by the pH of the composition. In one example, the pH of the anti-acne face cleansing composition is less than 5.0.

In some examples, the exfoliating anti-acne face cleansing composition is included in an anti-acne face cleansing product, in which the anti-acne face cleansing composition is included in a container. The container may be equipped with an opening capable of dispensing the exfoliating anti-acne face cleansing composition. An opening in the container dispenses the exfoliating anti-acne face cleansing composition by a plunger type mechanism, or another appropriate dispenser. The opening dispenses the exfoliating anti-acne face cleansing composition onto an area of the skin. For example, the opening may dispense the exfoliating anti-acne face cleansing composition onto a hand of a user. Another type of dispensing container includes a simple opening allowing for the dispensing of the exfoliating anti-acne face cleansing composition, which container may have a cap.

Table (1) gives an example of the anti-acne face cleansing composition as described in the present specification. More specifically, the anti-acne face cleansing composition of Table (1) includes the below listed ingredients in the indicated weight percentages.

**Table (1)**

| Ingredient | Weight Percent (%) |
|---|---|
| Water | 70-90 |
| Surfactant | 0.01 - 15.00 |
| Moisturizer | 1.00 - 10.00 |
| pH Adjuster | 0.01 - 2.00 |
| High Molecular Weight Polymer | 0.01 - 5.00 |
| Exfoliating Particle | 0.01 - 10.00 |
| Active Ingredient | 0.01 - 2.00 |
| Fragrance, Dyes, Extracts, Additives | 0.01 - 1.00 |

The exfoliating anti-acne face cleansing composition indicated in Table (1) may have a viscosity between 3,000 and 100,000 Centipoise (cps).Table (2) indicates different possible ratio ranges of components within the exfoliating anti-acne face cleansing composition of Table (1).

**Table (2)**

| Components | Ratio |
|---|---|
| High Molecular Weight Polymer:Moisturizers | 4:10 - 9:10 |
| High Molecular Weight Polymer:Surfactant | 1:10 - 2:10 |
| Surfactant:Moisturizer | 30:10 - 50:10 |
| Total Formula Solids:Moisturizers | 60:10 - 80:100 |
| High Molecular Weight Polymer:Total Formula Solids | 8:100 - 12:100 |

The exfoliating anti-acne face cleansing composition indicated in Tables (1) and (2) may have a viscosity between 5,000 and 100,000 Centipoise (cps).

As demonstrated above, the exfoliating anti-acne face cleansing composition may be unique in that the amount of the different components, and more specifically the ratios of the different components, lead to unexpectedly beneficial moisturization properties. Put another way, the exfoliating anti-acne face cleansing composition is defined by the moisturization properties, in addition to the above stated amounts of ingredients.

A face cleansing composition which is moisturizing, or which may be more moisturizing than water, may have increased consumer appeal as a result of its ability to increase the moisture content of the skin surface, which may make the skin surface appear softer and healthier. Water may be used as a threshold, and if a face cleansing composition demonstrates a statistical improvement to moisturization properties when compared to water, the face cleansing composition is said to be moisturizing. The moisturizing effect of a face cleansing composition may be measured by skin conductance, corneometry or visual evaluation. Each of skin conductance, corneometry and visual evaluation compare a baseline measurement with a number of measurements taken after treatment with a face cleansing composition, which may be exfoliating. The number of measurements taken after treatment with a face cleansing composition, which may be exfoliating, may be taken at regular intervals, are used as a basis of comparison between a number of face cleansing compositions.

Skin conductance is a method of measuring the moisture content of skin. Comparing values before treatment with a skin cleansing composition and at regular intervals following treatment allows for a determination of whether a face cleansing composition is moisturizing, or whether a face cleansing composition removes moisture from skin. Such a treatment includes washing with a face cleansing composition. Test washing with a face cleansing composition may be conducted on the lower leg, and skin conductance measurements may be made at the wash site. Temperature and humidity may affect skin conductance measurements, and so may be held constant from the baseline measurement to subsequent measurements. Skin conductance is measured in units of microSiemens, and is the reciprocal of skin resistance. Skin conductance measurements may be presented as the difference from the baseline measurement.

Table (3) below presents skin conductance measurements comparing the exfoliating anti-acne face cleansing composition to both water and a moisturizing skin cleansing composition, which serves as a non-exfoliating positive control, and be so marked in Table (3). The values presented in Table (3) indicate the difference from the baseline measurement, and represent the mean deviation from the baseline measurement for a study group of nineteen subjects. Positive numbers indicate an increase in the moisture content of the skin; negative numbers indicate a decrease in the moisture content of the skin. Values are presented as the difference from the baseline measurement, in units of microSiemens.

**Table (3)**

| | 0.5 hrs. | 1 hr. | 2 hrs. | 3 hrs. | Overall |
|---|---|---|---|---|---|
| Exfoliating Anti-Acne Face Cleansing Composition | 0.41 | -1.76 | -2.39 | -4.22 | -1.99 |
| Water | -1.62 | -3.62 | -3.30 | -6.03 | -3.64 |
| Positive Control | 5.46 | 0.80 | 1.63 | -1.51 | 1.60 |

As indicated in Table (3), the exfoliating anti-acne face cleansing composition may be more moisturizing than water. Since the measurements at each time show a larger negative number for water than for the exfoliating anti-acne face cleansing composition, and negative numbers indicate a decrease in the moisture content of the skin, the exfoliating anti-acne face cleansing composition may be said to be moisturizing. Accordingly, in some examples, the exfoliating face cleansing composition may cause decreases in an amount less than 5.0 microSiemens to skin conductance measurements of skin which has been treated with the exfoliating anti-acne face cleansing composition.

Corneometry is another way to measure the moisture content of skin. Corneometry may measure the capacitance of the skin, which may be a dielectric medium. The measured capacitance may be used to determine the dielectric constant of the skin, which may be converted to an arbitrary hydration unit, which may indicate a moisture content of the skin. Corneometry measures the capacitance and hydration of the stratum corneum, which is an outer layer of the epidermis. The stratum corneum serves as a barrier, and measurements of its hydration may be used as a proxy for its ability to function as a barrier.

Table (4) below presents corneometry measurements comparing the exfoliating anti-acne face cleansing composition to both water and a moisturizing skin cleansing composition, which serves as a non-exfoliating positive control, and be so marked in Table (4). The values presented in Table (4) are presented as the difference from the baseline measurement, and may represent the mean deviation from the baseline measurement for a study group of nineteen subjects. Positive numbers indicate an increase in the moisture content of the skin; negative numbers indicate a decrease in the moisture content of the skin. Values may be presented as the mean difference from the baseline measurement, in corneometry units.

**Table (4)**

| | 0.5 hrs. | 1 hr. | 2 hrs. | 3 hrs. | Overall |
|---|---|---|---|---|---|
| Exfoliating Anti-Acne Face Cleansing Composition | 1.09 | -0.14 | -0.49 | 0.86 | 0.33 |
| Water | -2.46 | -3.22 | -2.80 | -1.91 | -2.60 |
| Positive Control | 3.04 | 1.86 | 1.17 | 3.18 | 2.31 |

As indicated in Table (4), the exfoliating anti-acne face cleansing composition may be more moisturizing than water. Since the measurements at each time show a larger negative number for water than for the exfoliating anti-acne face cleansing composition, and negative numbers indicate a decrease in the moisture content of the skin, the exfoliating anti-acne face cleansing composition may be said to be moisturizing. Accordingly, in some examples, the exfoliating face cleansing composition may cause decreases in an amount less than 2.0 corneometry units to corneometer measurements of skin which has been treated with the face cleansing composition.

Moisture content of skin may also be determined by visual evaluations. Similar to both skin conductance and corneometry, visual evaluation may be relative to a baseline evaluation. Negative values for visual evaluation indicate an increase in the moisture content of the skin, which may be measured as a decrease in the visual evaluation of skin dryness. Visual dryness evaluations may be conducted by an evaluator using a 3X diopter lens illuminated magnifying glass. A baseline evaluation may be conducted, as well as subsequent evaluations at 0.5, 1, 2, and 3 hours after application. Evaluations may be assigned a numerical value, or 'grade,' according to the scale presented in Table (5).

**Table (5)**

| Grade | Dryness |
|---|---|
| 0.0 | No visible dryness |
| 0.5 | Perceptible dryness with fine white lines |
| 1.0 | Fine dry lines and a white powdery appearance, which may be accompanied by some uplifting flakes on less than 30% of the evaluation site |
| 1.5 | Uniform flaking which covers 30-50% of the evaluation site |
| 2.0 | Uniform, marked flaking covering more than 50% of the evaluation site |
| 2.5 | Slight to moderate scaling |
| 3.0 | Moderate to severe scaling, which may be accompanied by uplifting scales |
| 3.5 | Severe scaling, which may be accompanied by slight fissuring |
| 4.0 | Severe scaling and fissuring |

In some examples, the anti-acne face cleansing composition may be defined by the difference from a baseline visual evaluation of dryness. A positive difference indicates an increase in dryness of the skin, which corresponds to a decrease in moisture content of the skin, and negative numbers indicate a decrease in dryness of the skin, which corresponds to an increase in moisture content of the skin. Values are presented as the average difference in the grade from the baseline measurement, according to the grading shown in Table (5). In some examples, skin treated with the exfoliating anti-acne face cleansing composition exhibits a change from a baseline visual dryness measurement less than 0.1. In other words, the exfoliating anti-acne face cleansing composition may cause average changes in an amount less than 0.1 to visual assessment of skin dryness according to an incremented grading system.

Similarly, visual erythema evaluations may be conducted by an evaluator. Visual erythema assessments comprise a visual assessment of any increased redness in the skin, which may be indicative of irritation to the skin. Evaluations are assigned a numerical value, or 'grade,' according to the scale presented in Table (6). Visual assessments of erythema may be presented as the average difference from the baseline measurement in the study group of 39 subjects, and may be measured according to the grading system presented in Table (6). Positive values indicate an increase in visual assessment of erythema, and negative values indicate a decrease in visual assessment of erythema.

**Table (6)**

| Grade | Erythema | Dryness |
|---|---|---|
| 0.0 | None | None |
| 0.5 | Perceptible | Perceptible, whiteness in the lines of the skin |
| 1.0 | Mild, slight | Slight flaking or uplifting of flakes, which may be accompanied by a patchy or powdered appearance |
| 1.5 | Slight to moderate | Slight to moderate flaking or uplifting of flakes, which may be uniform on the evaluation site |
| 2.0 | Moderate, confluent | Moderate flaking or uplifting of flakes, which may be uniform on the evaluation site, and may also be accompanied by slight scaling |
| 2.5 | Moderate to marked | Moderate to severe flaking or uplifting of flakes, which may be accompanied by moderate scaling |
| 3.0 | Marked, prominent | Severe flaking or scaling, uplifting of scales, which may be accompanied by slight assuring |

In some examples, skin treated with the exfoliating anti-acne face cleansing composition exhibits a change from a baseline visual erythema measurement less than 0.2. In other words, the exfoliating anti-acne face cleansing composition may cause average changes in an amount less than 0.2 to visual assessment of skin erythema according to an incremented grading system.

While at least one exemplary embodiment has been presented in the foregoing detailed description of the invention, it should be appreciated that a vast number of variations exist. It should also be appreciated that the exemplary embodiment or exemplary embodiments are only examples, and are not intended to limit the scope, applicability, or configuration of the invention in any way. Rather, the foregoing detailed description will provide those skilled in the art with a convenient road map for implementing an exemplary embodiment of the invention, it being understood that various changes may be made in the function and arrangement of elements described in an exemplary embodiment without departing from the scope of the invention as set forth in the appended claims and their legal equivalents.

## Claims

1. An exfoliating anti-acne face cleansing composition, comprising:
an anti-acne active ingredient;
exfoliating particles;
a high molecular weight polymer having a molecular weight at least equal to ten kiloDaltons (kD); and
a moisturizer;
in which:
a ratio of the high molecular weight polymer to total formula solids is between 8:100 and 12:100;
a ratio of the high molecular weight polymer to an amount of moisturizer is between 4:10 and 9:10; and
a pH of the exfoliating anti-acne face cleansing composition is less than 5.0.

2. The exfoliating anti-acne face cleansing composition of claim 1, in which the anti-acne active ingredient is salicylic acid.

3. The exfoliating anti-acne face cleansing composition of claim 1, in which the high molecular weight polymer is a cross-linked copolymer of acrylic acid or methacrylic acid or a number of their simple esters.

4. The exfoliating anti-acne face cleansing composition of claim 1, further comprising a surfactant, a pH adjuster, a suspending agent, a fragrance, a dye, an extract, or combinations thereof.

5. The exfoliating anti-acne face cleansing composition of claim 4, in which a ratio of high molecular weight polymer to surfactant is between 1:10 and 2:10.

6. The exfoliating anti-acne face cleansing composition of claim 4, in which a ratio of surfactant to the amount of moisturizer is between 30:10 and 50:10.

7. The exfoliating anti-acne face cleansing composition of claim 1, in which a ratio of total formula solids to the amount of moisturizer is between 60:10 and 80:10.

8. An exfoliating anti-acne face cleansing product, comprising:
a container; and
an exfoliating anti-acne face cleansing composition disposed within the container, the exfoliating face cleansing composition comprising;
an anti-acne active ingredient;
exfoliating particles;
a high molecular weight polymer having a molecular weight of at least ten kiloDaltons (kD);
a surfactant;
a moisturizer; and
an aqueous medium in which the anti-acne active ingredient, the exfoliating particles, the high molecular weight polymer, the surfactant, and the moisturizer are distributed;
in which:
a ratio of the high molecular weight polymer to total formula solids is between 8:100 and 12:100;
a ratio of the high molecular weight polymer to an amount of moisturizer is between 4:10 and 9:10;
a pH of the exfoliating anti-acne face cleansing composition is less than 5.0.

9. The exfoliating anti-acne face cleansing product of claim 8, in which the exfoliating anti-acne face cleansing composition causes decreases in an amount less than 5.0 microSiemens to skin conductance measurements of skin which has been treated with the exfoliating anti-acne face cleansing composition.

10. The exfoliating anti-acne face cleansing product of claim 8, in which the exfoliating anti-acne face cleansing composition causes decreases in amount less than 2.0 corneometry units to corneometer measurements of skin which has been treated with the exfoliating anti-acne face cleansing composition.

11. The exfoliating anti-acne face cleansing product of claim 8, in which the exfoliating anti-acne face cleansing composition causes average changes in an amount less than 0.2 to visual assessments of erythema, according to an incremented grading system.

12. The exfoliating anti-acne face cleansing product of claim 8, in which the exfoliating anti-acne face cleansing composition causes average changes in an amount less than 0.1 to visual assessments of skin dryness, according to an incremented grading system.

13. The exfoliating anti-acne face cleansing product of claim 8, in which a ratio of high molecular weight polymer to surfactant is between 1:10 and 2:10.

14. The exfoliating anti-acne face cleansing product of claim 8, in which a ratio of surfactant to the amount of moisturizer is between 30:10 and 50:10.

15. An exfoliating anti-acne face cleansing composition, comprising:
salicylic acid;
exfoliating particles;
a surfactant;
a cross-linked copolymer of acrylic acid, a cross-linked copolymer of methacrylic acid, or combinations thereof; and
and a moisturizer;
in which:
a ratio of the cross-linked copolymer of acrylic acid, a cross-linked copolymer of methacrylic acid, or combinations thereof to total formula solids is between 8:100 and 12:100;
a ratio of the cross-linked copolymer of acrylic acid, a cross-linked copolymer of methacrylic acid, or combinations thereof to an amount of moisturizer is between 4:10 and 9:10; and
a pH of the exfoliating anti-acne face cleansing composition is less than 5.0.

16. The exfoliating anti-acne face cleansing composition of claim 15, wherein the ratio of the cross-linked copolymer of acrylic acid, a cross-linked copolymer of methacrylic acid, or combinations thereof to total formula solids is between 9:100 and 11:00.

17. The exfoliating anti-acne face cleansing composition of claim 15, wherein the ratio of the cross-linked copolymer of acrylic acid, a cross-linked copolymer of methacrylic acid, or combinations thereof to the amount of moisturizer is between 5:10 and 8:10.

18. The exfoliating anti-acne face cleansing composition of claim 15, in which the ratio the cross-linked copolymer of acrylic acid, a cross-linked copolymer of methacrylic acid, or combinations thereof to the surfactant is between 1.25:10 and 1.75:10.

19. The exfoliating anti-acne face cleansing composition of claim 15, in which the ratio the surfactant to the amount of moisturizer is between 40:10 and 45:10.

20. The exfoliating anti-acne face cleansing composition of claim 15, in which the ratio the total formula solids to the amount of moisturizer is between 65:10 and 80:10.
